# EUROPÄISCHE PATENTANMELDUNG

(11) **EP 0 567 881 A2**
(43) Veröffentlichungstag der Anmeldung: **03.11.1993**
(21) Anmeldenummer: 93106306.9
(22) Anmeldetag: 19.04.1993
(51) Int. Cl.: C07C 209/78, C07C 211/50

(54) **Verfahren zur Herstellung von Gemischen von Polyaminen der Polyamino-polyaryl-polymethylenreihe**

(30) Priorität: 01.05.1992 US 877684
(71) Anmelder: BAYER AG, 51368 Leverkusen (DE)
(72) Erfinder: König, Christian, Dr., W-4044 Kaarst (DE); Gallus, Manfred, Dr., W-4150 Krefeld (DE); Wüllner, Reinhold, Dr., W-4150 Krefeld (DE); Richter, Franz-Moritz, Dr., W-2222 Marne (DE); Uchdorf, Rudolf, Dipl.-Ing., W-4150 Krefeld 11 (DE); Forester, William C., Dipl.-Ing., Upter St. Clair, PA 15241 (US)

(57) **Zusammenfassung**

Die Erfindung betrifft ein verbessertes Verfahren zur Herstellung von Gemischen von Polyaminen der Polyamino-polyaryl-polymethylenreihe sowie deren Verwendung zur Herstellung weißer Polyurethanschaumstoffe.

## Beschreibung

Die Erfindung betrifft ein verbessertes Verfahren zur Herstellung von Gemischen von Polyaminen der Polyaminopolyaryl-polymethylenreihe sowie deren Verwendung zur Herstellung weißer Polyurethanschaumstoffe.

Die Herstellung von Diaminoarylmethanen und Gemischen von Methylenbrücken aufweisenden Polyarylpolyaminen, die die Diaminoarylmethane enthalten, durch saure Kondensation von aromatischen Aminen und Formaldehyd ist bekannt (siehe beispielsweise US-PS 2 683 730). Die Diaminoarylmethane und die Polyamingemische sind als Zwischenprodukte bei der Herstellung der entsprechenden Di- und Polyisocyanate wertvoll. Bei der Verschäumung dieser Di- und Polyisocyanate weisen die resultierenden Polyurethanschäume eine gelb-braune Färbung auf. Diese Färbung des Schaumes dient wiederum als Qualitätsmerkmal des eingesetzten Isocyanats. Je dunkler die Färbung desto schlechter ist das eingesetzte Isocyanat.

Die DE-A-32 25 135 beschreibt beispielsweise ein Verfahren zur Herstellung von Methylenbrücken aufweisenden Polyarylaminen, bei dem die Komponenten vorzugsweise bei Temperaturen bis 50°C gemischt werden und bei dem danach die Reaktionsmischung in einem Rohrreaktor unter kontinuierlicher Temperaturerhöhung von 50°C auf 90° bis 100°C zur Reaktion gebracht wird. Mit diesen Polyaminen hergestellte Polyurethanschaumstoffe sind dunkel verfärbt.

EP-A-14927 beschreibt ein spezielles Verfahren zur Herstellung von Polyamingemischen der Polyamino-polyaryl-polymethylenreihe, bei dem das Amin mit Formaldehyd in Gegenwart eines sauren Katalysators bei 10° bis 100°C umgesetzt wird bei einem Protonierungsgrad von 0,1 bis 25 %, anschließend nochmals Säure als Katalysator zugesetzt wird, anschließend die Temperatur auf 75° bis 150°C einstellt und danach die Polyamine isoliert werden. Nachteil dieses Verfahrens ist das aufwendige Splitting des sauren Katalysators. Zudem erhält man mit diesen Polyaminen ebenfalls keine hellen Polyurethanschäume.

Aufgabe war es daher, ein Verfahren zur Verfügung zu stellen, daß es gestattet, qualitativ hochwertige Isocyanate herzustellen, die einen hellen Polyurethanschaumstoff ergeben.

Diese Aufgabe konnte durch das erfindungsgemäße Verfahren gelöst werden.

Gegenstand der Erfindung ist ein Verfahren zur Herstellung von Polyaminen der Diphenylmethanreihe durch Kondensation von Anilin mit Formaldehyd, weitere Umsetzung in Gegenwart eines sauren Katalysators, Neutralisation des Säurekatalysators nach beendeter Reaktion und destillative Aufarbeitung des so erhaltenen Polyamingemisches, dadurch gekennzeichnet, daß man
a) Anilin mit Formaldehyd im Molverhältnis von 1,5 bis 10 bei Temperaturen von 10° bis 150°C kondensiert und gegebenenfalls das Reaktionswasser abtrennt,
b) anschließend dem Reaktionsgemisch aus a) einen sauren Katalysator zumischt, wobei das Molverhältnis von Anilin zu saurem Katalysator 2 bis 100 (entsprechend einem Protonierungsgrad von 1 bis 50 %) und die Temperatur 10° bis 150°C beträgt,
c) die so erhaltene Mischung aus b) in maximal 15 Minuten um mindestens 40°C erhitzt und anschließend gegebenenfalls weiter auf eine Endtemperatur von 105° bis 200°C aufheizt, wobei die Reaktionszeit nach der Temperaturerhöhung um mindestens 40°C 10 bis 300 Minuten beträgt und
d) das Reaktionsgemisch aus c) nach Neutralisation des sauren Katalysators destillativ aufarbeitet.

Das erfindungsgemäße Verfahren eröffnet einen Weg zur Herstellung von Polyamingemischen der Diphenylmethanreihe, die nach Phosgenierung und Verschäumung zu weißen Polyurethanschaumstoffen führen, die mit den bekannten Verfahren nicht herzustellen waren.

In einer besonderen Ausführungsform des erfindungsgemäßen Verfahrens wird nach Stufe a) oder b) dem Reaktionsgemisch weiterer Formaldehyd zugesetzt, wobei das mit beiden Zugaben erzielte Molverhältnis Anilin zu Formaldehyd 1,5 bis 5,0 beträgt.

Das erfindungsgemäße Verfahren kann auch so durchgeführt werden, daß
b) Anilin mit saurem Katalysator im Molverhältnis 2 bis 100 (entsprechend einem Protonierungsgrad von 1 bis 50 %) umgesetzt wird,
a) anschließend diesem Gemisch aus b) Formaldehyd zugemischt wird, wobei das Molverhältnis Anilin zu Formaldehyd 1,5 bis 10 und die Temperatur 10 bis 150°C beträgt und
anschließend die Reaktionsstufen c) und d) durchgeführt werden.

Das erfindungsgemäße Verfahren unterscheidet sich von den bisher bekannten Verfahren dadurch, daß beim erfindungsgemäßen Verfahren die Temperatur des Reaktionsgemisches nach dem Kondensationsschritt bzw. nach der ersten Umlagerung spontan, d.h. in weniger als 15 Minuten, um mindestens 40°C erhöht wird und anschließend die zweite Umlagerung zum Polyamin bei Endtemperaturen von 105° bis 200°C durchgeführt wird.

Überraschenderweise hat sich gezeigt, daß die erfindungsgemäße Temperaturführung einen erheblichen positiven Einfluß auf die Qualität des so hergestellten Polyamingemisches hat und damit auf den daraus hergestellten Polyurethanschaumstoff.

Auch ist die Reaktionszeit nach der Temperaturerhöhung um mindestens 40°C höher als in bekannten Verfahren, wobei bisher davon ausgegangen wurde, daß eine Reaktionszeitverlängerung bei erhöhter Temperatur "bei der zweiten Umlagerung" zu schlechten Polyaminen führt, die dunklere Polyurethanschaumstoffe ergeben.

Bevorzugt wird das erfindungsgemäße Verfahren so durchgeführt, daß die Temperatur der Stufe a) 50 bis 120°C beträgt und das Molverhältnis Anilin zu Formaldehyd in der Stufe a) 1,5 bis 4,0 beträgt.

Als Katalysator wird vorzugsweise Salzsäure eingesetzt.

In der Stufe b) ist das Molverhältnis Anilin zu saurem Katalysator bevorzugt 2 bis 20 (entsprechend einem Protonierungsgrad von 5 bis 50 %).

In einer bevorzugten Ausführungsform ist die Temperatur vor der Stufe c) 35° bis 60°C und die Endtemperatur der Stufe c) 150° bis 180°C.

Die nach dem erfindungsgemäßen Verfahren hergestellten Polyamingemische werden nach Phosgenierung zu Polyisocyanatgemischen als Ausgangsmaterial zur Herstellung von hellen Polyurethanschaumstoffen verwendet.

Es ist allgemein bekannt, daß die saure Kondensation von aromatischen Aminen und Formaldehyd zu Verbindungen der Diaminodiphenylmethan-Reihe in mehreren Reaktionsschritten abläuft:

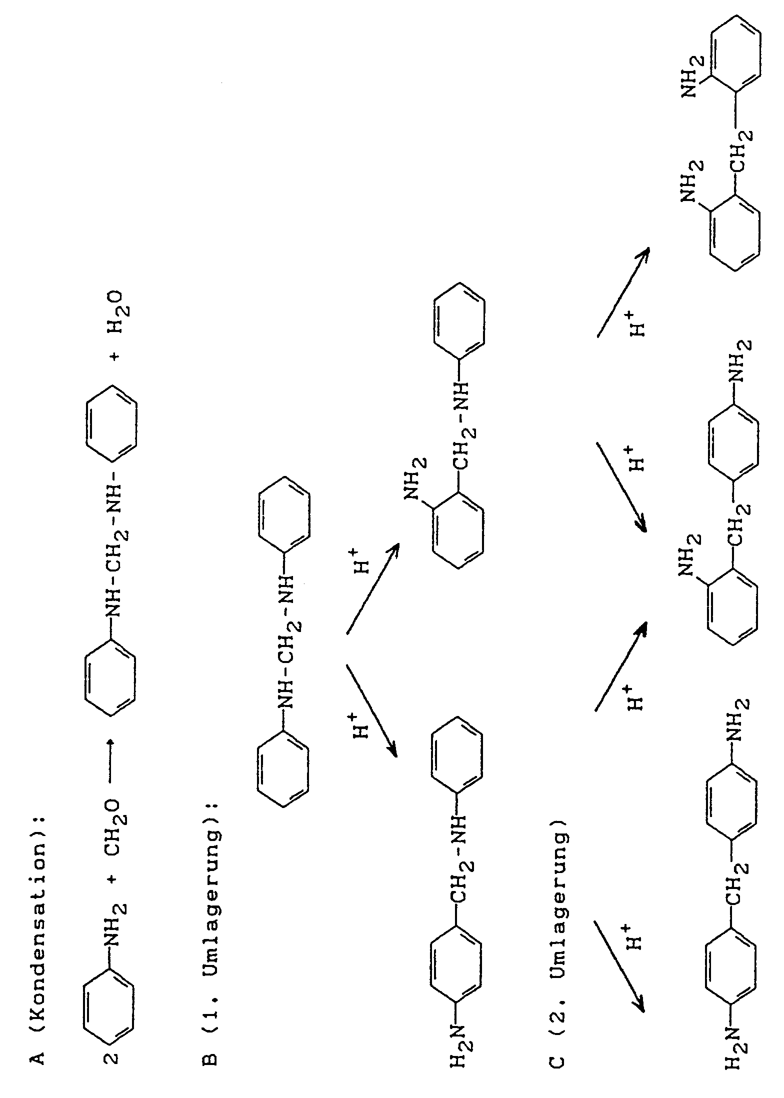

Bei der Durchführung des erfindungsgemäßen Verfahrens erfolgt die Kondensation im allgemeinen durch Zusammenführung von Anilin und wäßriger Formaldehyd-Lösung sowie gegebenenfalls anschließendem Abtrennen der gebildeten wäßrigen Phase. Die Reaktion zwischen dem Anilin und Formaldehyd ist exotherm. Gegebenenfalls kann das Reaktionsgemisch gekühlt werden. Die Reaktion findet bei einer Temperatur von 10 bis 150°C, vorzugsweise von 50 bis 120°C, statt. Das Molverhältnis von Anilin zu Formaldehyd bestimmt den Anteil an Diaminodiphenylmethan-Verbindungen im Amingemisch. Es beträgt 1,5 bis 10 zu 1, vorzugsweise 1,5 bis 4,0 zu 1.

Das Kondensat der genannten Reaktionsstufe wird anschließend in Stufe b) mit einem sauren Katalysator, vorzugsweise einer wäßrigen HCl-Lösung, versetzt. Auch diese Reaktion ist exotherm und kann durch Kühlung auf einen gewünschten Temperaturwert eingestellt werden. Dabei liegt die Temperatur im Bereich von 10° bis 150°C, vorzugsweise von 35° bis 60°C. Das Molverhältnis Anilin zu saurem Katalysator bestimmt den Anteil von o- und p-Substituenten im Endprodukt. Das Molverhältnis Anilin zu Katalysator liegt zwischen 2:1 bis 100:1, vorzugsweise 2:1 bis 20:1. Dieses Verhältnis kann auch als Protonierungsgrad definiert werden. Der Protonierungsgrad gibt an, wieviel Prozent des eingesetzten Anilins in Form von Ammoniumsalzen, also in protonierter Form, vorliegen. Der Protonierungsgrad liegt also zwischen 1 % und 50 %, vorzugsweise zwischen 5 % und 50 %. Es ist für das Verfahren unerheblich, ob das in Stufe a) abgeschiedene Reaktionswasser dem System entzogen wird oder ob es in die Stufe b) eingetragen wird.

Die Zusammenführung der Einsatzstoffe Anilin, Formalin und saurer Katalysator kann auch in einer Stufe erfolgen, wobei üblicherweise zunächst das Anilinsalz gebildet wird und dieses Gemisch mit Formaldehyd zur Reaktion gebracht wird. Die Mengenverhältnisse Anilin zu Formaldehyd zu saurem Katalysator sowie die Temperaturen sind dieselben wie oben beschrieben.

Weiterhin kann vor, während oder nach der in Stufe b) beschriebenen Einspeisung des sauren Katalysators weiteres Formaldehyd zugesetzt werden. Das Gesamtmolverhältnis Anilin zu Formaldehyd beträgt nach der gesamten Formaldehydzugabe 1,5 bis 5,0 zu 1.

Die Temperaturerhöhung um mindestens 40°C soll in maximal 15 Minuten erfolgen. Daran kann sich gegebenenfalls eine Temperaturerhöhung auf 105° bis 200°C, vorzugsweise auf 150° bis 180°C anschließen. Die Reaktionszeit nach dem Erhitzen sollte 10 bis 300 Minuten betragen in Abhängigkeit vom Protonierungsgrad der sauren Reaktionslösung und damit von der Reaktionsgeschwindigkeit. Das saure Reaktionsgemisch wird anschließend neutralisiert und das Polyamin durch Destillation von überschüssigem Anilin befreit.

Die Phosgenierung des Polyamingemisches erfolgt nach bekannten technischen Verfahren.

Der Polyurethanschaum wird ebenfalls nach bekannten Verfahren hergestellt.

Zur Beurteilung der Färbung des Schaumstoffes wird die Extinktion des Isocyanates bei 430 nm sowie bei 520 nm herangezogen. Erfahrungsgemäß zeigt der Wert bei 430 nm die Gelb-Braun-Färbung an und der Wert bei 520 nm die Grau-Färbung des Schaumes. Niedrige Extinktionswerte zeigen an, daß die Verfärbung des Schaumes hell sein wird.

Die Erfindung soll anhand der nachfolgenden Beispiele näher erläutert werden.

### Beispiel 1 (Vergleichsbeispiel)

7060 kg/h Anilin und 2970 kg/h Formalin (32 %ige wäßrige Lösung) wurden gemischt und bei 80°C kondensiert (Mol-Verhältnis Anilin zu Formaldehyd = 2,3:1). Nach Abscheidung des Reaktionswassers wurden dem Reaktionsgemisch in einem 8 m³ Rührbehälter 2215 kg/h HCl (30 %ige wäßrige Lösung) zugeführt (Protonierungsgrad = 25 %) und die Temperatur durch Vakuumkühlung auf 40°C gehalten. Die so erhaltene Mischung wurde in fünf nachfolgenden Reaktionsrührbehältern (je 8 m³) allmählich mit folgenden Temperaturabstufungen aufgeheizt: 55°-75°-105°-115°-120°C. Das Gemisch wurde mit Natronlauge neutralisiert, von der wäßrigen Salzlösung abgetrennt und destillativ von restlichem Wasser und überschüssigem Anilin befreit. Das erhaltene Polyamin bestand aus 64,0 Gew.-% Diaminodiphenylmethan und 36,0 Gew.-% höheren Oligomeren. Nach der Phosgenierung des Polyamins enthält das Isocyanatgemisch ca. 50,0 Gew`-% Diisocyanatodiphenylmethan. Die Extinktionswerte dieses Isocyanates, die mit der späteren Färbung des Polyurethanschaumes korrelieren, betrugen bei 430 nm 0,275 und bei 520 nm 0,046.

### Beispiel 2 (erfindungsgemäß)

7060 kg/h Anilin und 2970 kg/h Formalin (32 %ige wäßrige Lösung) wurden gemischt und bei 80°C kondensiert (Mol-Verhältnis Anilin zu Formaldehyd = 2,3:1). Nach Abscheidung des Reaktionswassers wurden dem Reaktionsgemisch in einem 8 m³ Rührbehälter 2215 kg/h HCl (30 %ige wäßrige Lösung) zugeführt (Protonierungsgrad = 25 %) und die Temperatur durch Vakuumkühlung auf 40°C gehalten. Die so erhaltene Mischung wurde im folgenden Reaktionsbehälter (8 m³) spontan auf 90°C erwärmt und in vier nachfolgenden Rührbehältern (je 8 m³) auf folgende Temperaturen hochgeheizt: 105°-150°-160°-160°C. Das Gemisch wurde mit Natronlauge neutralisiert, von der wäßrigen Salzlösung abgetrennt und destillativ vom restlichen Wasser und überschüssigem Anilin befreit. Das erhaltene Polyamin bestand aus 64,1 Gew.-% Diaminodiphenylmethan und 35,9 Gew.-% höheren Oligomeren.

Nach der Phosgenierung des Polyamins enthielt das Isocyanat-Gemisch ca. 50,0 Gew.-% Diisocyanatodiphenylmethan.

Die Extinktionswerte dieses Isocyanates, die mit der späteren Färbung des Polyurethanschaums korrelieren, betrugen bei 430 nm 0,068 und bei 520 nm 0,014.

### Beispiel 3 (Vergleichsbeispiel)

5900 kg/h Anilin und 3000 kg/h Formalin (32 %ige wäßrige Lösung) werden gemischt und bei 80°C kondensiert (Mol-Verhältnis Anilin zu Formaldehyd 1,9:1). Nach Abscheidung des Reaktionswassers werden dem Reaktionsgemisch in einem 8 m³ Rührbehälter 1850 kg/h HCl (30 %ige wäßrige Lösung) zugeführt (Protonierungsgrad = 25 %) und die Temperatur durch Vakuumkühlung auf 40°C gehalten.

Die so erhaltene Mischung wurde in den fünf nachfolgenden Reaktionsbehältern (je 8 m³) in folgenden Temperaturabstufungen aufgeheizt: 55°-70°-140°-150°-150°C. Das Gemisch wurde mit Natronlauge neutralisiert, von der wäßrigen Salzlösung abgetrennt und destillativ von restlichem Wasser und überschüssigem Anilin befreit. Das erhaltene Polyamin bestand aus 51,0 Gew.-% Diaminodiphenylmethan und 49,0 Gew.-% höheren Oligomeren.

Nach der Phosgenierung des Polyamins enthielt das Isocyanat-Gemisch ca. 50,0 Gew.-% Diisocyanatodiphenylmethan. Die Extinktionswerte dieses Isocyanates, die mit der späteren Färbung des Polyurethanschaumes korrelieren, betrugen bei 430 nm 0,207 und bei 520 nm 0,056.

### Beispiel 4 (erfindungsgemäß)

Bei ansonsten gleichen Versuchsbedingungen wie in Beispiel 3 wurden hinter dem mit 40°C betriebenen Reaktor die Temperatur in nachfolgendem Rührkessel spontan auf 90°C angehoben. Die weiteren Temperaturen in den vier nachfolgenden Reaktoren betrugen: 105°-140°-150°-150°C.

Anschließend wurde wie in Beispiel 3 verfahren. Die Extinktionswerte dieses Isocyanates betrugen bei 430 nm 0,105 und bei 520 nm 0,028.

## Patentansprüche

1. Verfahren zur Herstellung von Polyaminen der Diphenylmethanreihe durch Kondensation von Anilin mit Formaldehyd, weitere Umsetzung in Gegenwart eines sauren Katalysators, Neutralisation des Säurekatalysators nach beendeter Reaktion und destillative Aufarbeitung des so erhaltenen Polyamingemisches, dadurch gekennzeichnet, daß man
a) Anilin mit Formaldehyd im Molverhältnis von 1,5 bis 10 bei Temperaturen von 10° bis 150°C kondensiert und gegebenenfalls das Reaktionswasser abtrennt,
b) anschließend dem Reaktionsgemisch aus a) einen sauren Katalysator zumischt, wobei das Molverhältnis von Anilin zu saurem Katalysator 2 bis 100 (entsprechend einem Protonierungsgrad von 1 bis 50 %) und die Temperatur 10° bis 150°C beträgt,
c) die so erhaltene Mischung aus b) in maximal 15 Minuten um mindestens 40°C erhitzt und anschließend gegebenenfalls weiter auf eine Endtemperatur von 105° bis 200°C aufheizt, wobei die Reaktionszeit nach der Temperaturerhöhung um mindestens 40°C 10 bis 300 Minuten beträgt und
d) das Reaktionsgemisch aus c) nach Neutralisation des sauren Katalysators destillativ aufarbeitet.

2. Verfahren nach Anspruch 1, dadurch gekennzeichnet, daß nach Stufe a) oder b) dem Reaktionsgemisch weiteres Formaldehyd zugesetzt wird, wobei das mit beiden Zugaben erzielte Molverhältnis Anilin zu Formaldehyd 1,5 bis 5,0 beträgt.

3. Verfahren nach Anspruch 1, dadurch gekennzeichnet, daß man zuerst
b) Anilin mit saurem Katalysator im Molverhältnis 2 bis 100 (entsprechend einem Protonierungsgrad von 1 bis 50 %) umsetzt und dann
a) dem Gemisch aus b) Formaldehyd zumischt, wobei das Molverhältnis Anilin zu Formaldehyd 1,5 bis 10 und die Temperatur 10 bis 150°C beträgt.

4. Verfahren nach einem der Ansprüche 1 bis 3, dadurch gekennzeichnet, daß die Temperatur der Stufe a) 50° bis 120°C beträgt.

5. Verfahren nach einem der Ansprüche 1 bis 3, dadurch gekennzeichnet, daß das Molverhältnis Anilin zu Formaldehyd in der Stufe a) 1,5 bis 4,0 beträgt.

6. Verfahren nach einem der Ansprüche 1 bis 3, dadurch gekennzeichnet, daß in der Stufe b) als Katalysator Salzsäure eingesetzt wird.

7. Verfahren nach einem der Ansprüche 1 bis 3, dadurch gekennzeichnet, daß in der Stufe b) das Molverhältnis Anilin zu saurem Katalysator 2 bis 20 (entsprechend einem Protonierungsgrad von 5 bis 50 %) beträgt.

8. Verfahren nach einem der Ansprüche 1 bis 3, dadurch gekennzeichnet, daß die Temperatur vor der Stufe c) 35° bis 60°C beträgt.

9. Verfahren nach einem der Ansprüche 1 bis 3, dadurch gekennzeichnet, daß die Endtemperatur der Stufe c) 150° bis 180°C beträgt.

10. Verwendung der nach einem der Verfahren gemäß den Ansprüchen 1 bis 9 erhaltenen Polyamingemische als Ausgangsmaterial zur Herstellung von hellen Polyurethanschaumstoffen.
